(19) Europäisches Patentamt
European Patent Office
Office européen des brevets

(11) Publication number: **0 376 518 B1**

# (12) EUROPEAN PATENT SPECIFICATION

(45) Date of publication of patent specification: **29.11.95**

(51) Int. Cl.[6]: **C07H 19/10**, C07H 19/20, A61K 31/70

(21) Application number: **89312830.6**

(22) Date of filing: **08.12.89**

(54) **Phospholipid nucleosides.**

(30) Priority: **12.12.88 US 282766**

(43) Date of publication of application:
**04.07.90 Bulletin 90/27**

(45) Publication of the grant of the patent:
**29.11.95 Bulletin 95/48**

(84) Designated Contracting States:
**DE ES FR GB IT**

(56) References cited:
**EP-A- 0 184 365**
**EP-A- 0 211 354**
**EP-A- 0 262 876**

**CHEMICAL ABSTRACTS, vol. 109, no. 15, 10th October 1988, page 25, abstract no. 122089a, Columbus, Ohio, US; V. HEINEMANN et al.: "Comparison of the cellular pharmacokinetics and toxicity of 2',2'-difluorodeoxycytidine and 1-beta-D- arabinofuranosylcytosine", & CANCER RES. 1988, 48(14), 4024-31**

(73) Proprietor: **ELI LILLY AND COMPANY**
**Lilly Corporate Center**
**Indianapolis**
**Indiana 46285 (US)**

(72) Inventor: **Bonjouklian, Rosanne**
**318 Dominion Drive**
**Zionsville**
**Indiana 46077 (US)**
Inventor: **Grindey, Gerald Burr**
**5223 East 77th Street**
**Indianapolis**
**Indiana 46250 (US)**
Inventor: **Hertel, Larry Wayne**
**2313 Ouiet Court**
**Indianapolis**
**Indiana 46239 (US)**

(74) Representative: **Hudson, Christopher Mark et al**
**Lilly Industries Limited**
**European Patent Operations**
**Erl Wood Manor**
**Windlesham**
**Surrey GU20 6PH (GB)**

EP 0 376 518 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**JOURNAL OF MEDICINAL CHEMISTRY, vol. 25, no. 11, 1982, pages 1322-1329, Columbus, Ohio, US; E.K. RYU et al.: "Phospholipid-nucleoside conjugates. 3. Syntheses and preliminary biological evaluation of 1-beta-D-arabinofuranosylcytosine 5'-monophosphate-L-1,2-dipalmitin and selected 1-beta- D-arabinofuranosylcytosine 5'-diphosphate-L-1,2-diacylglycerols"**

## Description

This invention belongs to the fields of pharmaceutical chemistry, synthetic organic chemistry and medicine, and provides new phospholipid derivatives of certain difluoronucleosides.

For some years, it has been known that some nucleosides have utility as anticancer agents, as well as antiviral agents. A series of 2'-deoxy-2',2'-difluoronucleosides were disclosed by Hertel, U.S. Patents 4,526,998 and 4,692,434. Those difluoronucleosides have now been modified to provide the phospholipid derivatives of the present invention.

Phospholipid-nucleosides with an unmodified ribose or arabinose are disclosed in E. K. Ryu et al. J. Med. Chem. 25, 1322 (1982) and EP-A-0262876.

The present invention provides the phospholipid derivatives of the formula

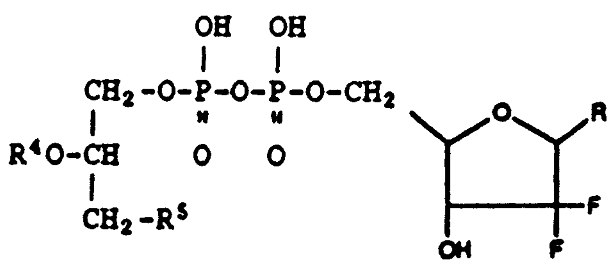

I

wherein R is a base of one of the formulae

A.

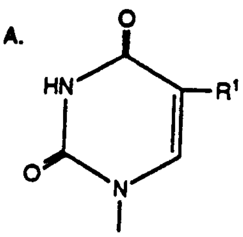

B.

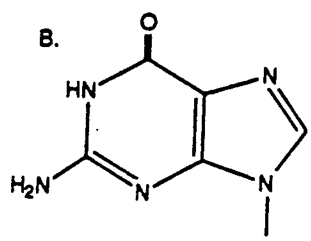

C.

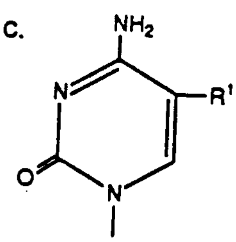

D.

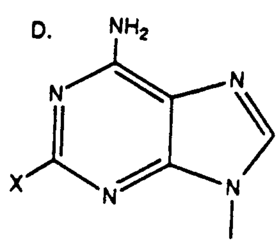

E.

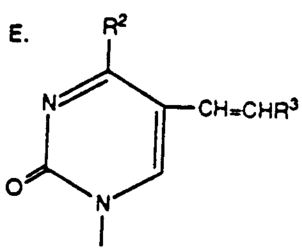

$R^1$ is hydrogen, $C_1$-$C_3$ alkyl, bromo, fluoro, chloro or iodo;
$R^2$ is hydroxy or amino;
$R^3$ is hydrogen, bromo, chloro or iodo;
X is hydrogen, amino, hydroxy, bromo, fluoro or chloro;

$R^4$ is $C_1$-$C_3$ alkyl or $R^6$;
$R^6$ is $CO\text{-}(CH_2)_mCH_3$;
m is an integer from 12 through 18;
$R^5$ is $(CH_2)_nCH_3$ or $R^7$;
$R^7$ is $O\text{-}CO\text{-}(CH_2)_mCH_3$;
n is an integer from 14 through 20;
provided that $R^4$ is $C_1$-$C_3$ alkyl when $R^5$ is $(CH_2)_nCH_3$;and that $R^4$ is $R^6$ when $R^5$ is $R^7$;
or a pharmaceutically acceptable salt thereof.

Also provided is a process for preparing the compounds of Formula I comprising reacting an intermediate of Formula II (below) in activated form with a phospholipid intermediate of the formula

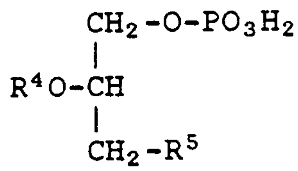

IV

wherein $R^4$ and $R^5$ are as defined above.

The invention also comprises pharmaceutical formulations comprising the compounds of Formula I and pharmaceutically-acceptable carriers, and methods of treating susceptible neoplasms in mammals comprising administering to a mammal in need of such treatment an effective amount of a compound of Formula I.

The present invention also provides a compound of the formula

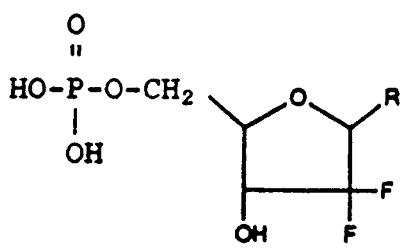

II

provided that when R is of formula C, above, $R^1$ is not hydrogen.

Further, the invention provides a process for preparing the compounds of Formula II by reacting a nucleoside of the formula

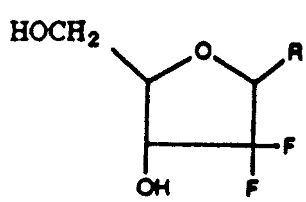

III

with a large excess of $POCl_3$ in the presence of a tri-$C_1$-$C_3$-alkylphosphate.

In this document, all temperatures are reported in degrees Celsius. All expressions of percentage, concentration and the like are in weight units unless otherwise stated.

The formulae above do not indicate the stereochemistry of the compounds of the present invention, and the stereochemistry is not a critical aspect of the invention. Accordingly, it will be understood that the present document refers to compounds of all possible stereochemical orientations, while recognizing that it is probable that certain stereochemical species will be found to be more effective than are other orientations.

For example, it has been found that the $\beta$-species of the difluoronucleosides of formula III are more efficacious than are the $\alpha$-species. The $\beta$-difluoronucleosides are therefore preferred starting compounds for

making the present phospholipid derivatives, and the corresponding β products of formula I are likewise preferred.

The difluoronucleosides of formula III are described by Hertel, U.S. Patent 4,692,434. The reader is invited to refer to that patent for the description of the starting compounds, for the manner of their synthesis, and for instances of preferred difluoronucleosides. It should be mentioned, however, that the most preferred difluoronucleoside is 2′-deoxy-2′,2′-difluorocytidine, which is prepared in Example 8 of that patent.

It is believed that the above formulae are entirely clear, and that an organic or pharmaceutical chemist can immediately recognize the compounds which are included in the present invention. However, to assure understanding, a number of exemplary compounds will be shown in the table below. The identity of the compounds is shown by use of the various symbols in the formulae, since that method of identification is believed to be easier to follow than is nucleoside nomenclature.

TABLE 1

| | R | $R^1$ | $R^2$ | $R^3$ | X | $R^4$ | m | $R^5$ | n |
|---|---|---|---|---|---|---|---|---|---|
| 1. | A | H | -- | -- | -- | $CH_3$ | -- | $(CH_2)_nCH_3$ | 14 |
| 2. | A | $CH_3$ | -- | -- | -- | $R^6$ | 12 | $R^7$ | -- |
| 3. | A | Br | -- | -- | -- | $n-C_3H_7$ | -- | $(CH_2)_nCH_3$ | 15 |
| 4. | A | F | -- | -- | -- | $C_2H_5$ | -- | $(CH_2)_nCH_3$ | 16 |
| 5. | A | Cl | -- | -- | -- | $i-C_3H_7$ | -- | $(CH_2)_nCH_3$ | 18 |
| 6. | A | I | -- | -- | -- | $C_2H_5$ | -- | $(CH_2)_nCH_3$ | 17 |
| 7. | A | $C_2H_5$ | -- | -- | -- | $R^6$ | 15 | $R^7$ | -- |
| 8. | A | $n-C_3H_7$ | -- | -- | -- | $R^6$ | 16 | $R^7$ | -- |
| 9. | A | $i-C_3H_7$ | -- | -- | -- | $C_2H_5$ | -- | $(CH_2)_nCH_3$ | 17 |
| 10. | B | -- | -- | -- | -- | $C_2H_5$ | -- | $CH_2)_nCH_3$ | 20 |
| 11. | B | -- | -- | -- | -- | $R^6$ | 13 | $R^7$ | -- |
| 12. | B | -- | -- | -- | -- | $R^6$ | 15 | $R^7$ | -- |
| 13. | C | H | -- | -- | -- | $R^6$ | 14 | $R^7$ | -- |
| 14. | C | $CH_3$ | -- | -- | -- | $R^6$ | 16 | $R^7$ | -- |
| 15. | C | Br | -- | -- | -- | $R^6$ | 18 | $R^7$ | -- |
| 16. | C | F | -- | -- | -- | $n-C_3H_7$ | -- | $(CH_2)_nCH_3$ | 14 |
| 17. | C | Cl | -- | -- | -- | $C_2H_5$ | -- | $(CH_2)_nCH_3$ | 16 |
| 18. | C | I | -- | -- | -- | $R^6$ | 14 | $R^7$ | -- |

TABLE 1 CONTINUED

| | R | $R^1$ | $R^2$ | $R^3$ | X | $R^4$ | m | $R^5$ | n |
|---|---|---|---|---|---|---|---|---|---|
| 19. | D | -- | -- | -- | H | $CH_3$ | -- | $(CH_2)_nCH_3$ | 19 |
| 20. | D | -- | -- | -- | F | $C_2H_5$ | -- | $(CH_2)_nCH_3$ | 14 |
| 21. | D | -- | -- | -- | OH | $R^6$ | 14 | $R^7$ | -- |
| 22. | D | -- | -- | -- | Cl | $R^6$ | 16 | $R^7$ | -- |
| 23. | D | -- | -- | -- | Br | $CH_3$ | -- | $(CH_2)_nCH_3$ | 15 |
| 24. | D | -- | -- | -- | $NH_2$ | $R^6$ | 18 | $R^7$ | -- |
| 25. | E | -- | OH | H | -- | $C_2H_5$ | -- | $(CH_2)_nCH_3$ | 15 |
| 26. | E | -- | $NH_2$ | Br | -- | $R^6$ | 17 | $R^7$ | -- |
| 27. | E | -- | $NH_2$ | Cl | -- | $CH_3$ | -- | $(CH_2)_nCH_3$ | 16 |
| 28. | E | -- | OH | I | -- | $i-C_3H_7$ | -- | $(CH_2)_nCH_3$ | 14 |

Certain classes of the compounds of the present invention are particularly preferred. The following list sets out preferred definitions of the different variables in the compounds. Preferred compounds, it will be understood, are prepared by choosing the preferred group. Further preferred compounds are obtained by combining preferred definitions to create further, more limited preferred groups.

1. R is base C;
2. R is base A, C or E;

3. R is base B or D;
4. R is base A or C;
5. $R^1$ is hydrogen;
6. $R^4$ is $C_1$-$C_3$ alkyl and $R^5$ is $(CH_2)_nCH_3$;
7. $R^4$ is $C_1$-$C_2$ alkyl and n is 15-19;
8. $R^4$ is $C_2$-$C_3$ alkyl and n is 16-18;
9. $R^4$ is $R^6$, $R^5$ is $R^7$ and m is 13-17;
10. $R^4$ is $R^6$, $R^5$ is $R^7$ and m is 14-16;
11. The compounds are in the $\beta$ form;
12. The compounds are salts;
13. The compounds are free bases;
14. The compounds are hydrohalides.

It will be understood that the phosphorous-containing moieties of the compound may actually be in forms other than as written. Thus, as written, each phosphorous atom bears an OH and a =O, but the hydrogen may be missing, forming an anion, or may be replaced by a salt-forming group, with no effect on the usefulness of the resulting compound. Such salt-forming moieties are chosen from those well known to form salts with acidic substances, and include, for example, alkali and alkaline earth metal ions, amines and quaternary ammonium groups. For example, potassium, lithium, sodium, calcium and the like ions may form such salts, as may diethylamine, diethanolamine, methylamine, tetraethylammonium, benzyltrimethylammonium, tetrabutylammonium and the like. It is preferred, however, that the phosphorous-containing groups be in the acid or anion form, and that such salts not be formed.

On the other hand, it is convenient and often desirable to form pharmaceutically acceptable salts by reaction between the bases of the compounds and acidic substances. Hydrohalides, particularly the hydrochloride and hydrobromide, are preferred such salts. As usual in pharmaceutical chemistry, however, it is entirely practical to form pharmaceutically acceptable acid addition salts with a wide range of mineral and organic acids, including phosphoric, sulfuric, toluenesulfonic, acetic, oxalic, glyoxylic, maleic, malonic, propionic, formic, nitric, nitrous, methanesulfonic, and the like acids.

The phosphate intermediates are prepared from Hertel's difluoronucleosides, cited above. Since the compounds of the present invention are made from the phosphate intermediates, the discussion above relating to the nucleoside portion of the compounds is also applicable to the phosphate intermediates. The preferred phosphate intermediates, thus, are those which are used to prepare the preferred compounds, and there is no need to discuss the phosphate intermediates separately.

The starting compounds for the synthesis of the present phospholipid derivatives are available, or are easily prepared with the assistance of information in the art. The ultimate starting compound is a difluoronucleoside as taught by the Hertel patents, cited above. It will be noted that the substituents on the base portions of the nucleosides are described more broadly here than in the Hertel patents. The fact makes no difference, synthetically, since the manner of making the nucleosides, and their use in making the present compounds, is the same. The starting compound may be in the free base form, or in the salt form; it is believed to be preferred to use a hydrohalide, most preferably the hydrochloride, of the difluoronucleoside.

That starting compound is reacted with $POCl_3$ in the presence of a trialkyl phosphate. Preferably, the new process of the present invention is used, wherein a large excess of the phosphoryl chloride is supplied to the reaction mixture. In that aspect of the invention, excess amounts of phosphoryl chloride in the range of from about 10 to about 50 times the stoichiometric amount, based on the nucleoside, are used.

The reaction with phosphoryl chloride provides the 5′-phosphate of formula II, and produces only an insignificant amount of the undesired 3′-phosphate. The reaction, thus, is highly selective, and proceeds in excellent yield in short periods of time. The reaction is carried out at moderately low temperatures, in the range of about -10° to about 20°, preferably close to 5°. It is preferred to use the trialkylphosphate, preferably trimethylphosphate, as the solvent in the reaction mixture.

Use of a large excess of phosphoryl chloride results in a considerable amount of inorganic residue in the reaction mixture, when the reaction has gone to essential completion. As the examples below demonstrate, the intermediate of formula II may be conveniently separated from the inorganic residue by chromatography, or, more simply, by repeated slurrying of the residue alternately with methanol and absolute ethanol.

The 5′-phosphate of formula II must be converted to an activated form in order to obtain efficient reaction in the next step. Groups commonly used for the activation of phosphate esters are effective here, and the preferred activating ester group is morpholine. Reaction with the activating ester moieties, including morpholine, is conveniently obtained by using a coupling reagent such as a carbodiimide, particularly, for

example, dicyclohexylcarbodiimide. The reaction can be carried out readily in convenient inert solvents, particularly aqueous alkanol, at moderately elevated temperatures. The most convenient temperature is the reflux temperature at ambient pressure. The resulting morpholine ester of the 5′-phosphate nucleoside can be expected to be in the form of a 1:1 complex with dicyclohexylcarbodiimide and morpholine. The complex form has no effect on the following reaction and may be carried on without resulting interference.

The final step of the process is to react the activated nucleoside 5′-phosphate with the phospholipid intermediate of the formula

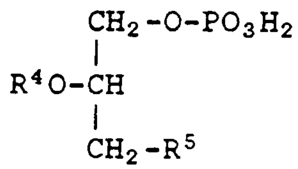

IV

The intermediates of formula IV of the bisalkanoyl type, wherein $R^4$ is $R^6$ and $R^5$ is $R^7$, are available from processing of naturally occurring fatty acids. Preparation of such bisalkanoylphosphatidic acids is in the literature, for example, Turcotte et al., Biochem. Biophys. Acta. 619, 604-18 (1980). The dialkyl intermediates of formula IV, where both $R^4$ and $R^5$ are alkyl, may be conveniently prepared from the chlorophosphates of Bonjouklian et al., U.S. Patent 4,659,859. The preparation below shows the conversion of that patent's chlorophosphates back to the phosphatidic acid of formula IV by simple hydrolysis, for example, with pyridine in an aqueous reaction mixture.

Reaction of the activated 5′-phosphate ester and the phosphatidic acid of formula IV is accomplished readily in a basic reaction medium, of which pyridine is preferred. The reaction goes nicely at ambient temperature and pressure, but can be carried out, at will, at moderate temperatures in the range from about 0° to about 50°. Inert organic solvents may be used in the reaction mixture, if desired, for example, to reduce the cost of the reaction mixture, but it is preferred to carry out the reaction in neat pyridine or other strong organic base which is a liquid at ambient conditions.

Isolation of the product is carried out by methods conventional in pharmaceutical chemistry, such as chromatography over silica gel. The examples below demonstrate the isolation and purification, and show that it is readily accomplished.

The following preparations and examples further illustrate the synthesis and compounds of the present invention.

Example 1

2′-Deoxy-2′,2′-difluorocytidine, 5′-dihydrogen phosphate

To a 100 ml flask containing 10 ml of trimethylphosphate and 6.75 ml of phosphoryl chloride at 5° was added 500 mg of 2′-deoxy-2′,2′-difluorocytidine, hydrochloride, in small portions with rapid stirring. The temperature was held constant and the mixture was continually stirred for about two hours. Small aliquots of the reaction mixture were removed from time to time, added to a mixture of aqueous sodium bicarbonate and diethyl ether, and nucleoside species in the aqueous layer were monitored by high performance liquid chromatography on a DuPont Zorbax ODS column, eluting with 1:9 methanol:aqueous ammonium acetate and observing the effluent at 254 nm, to follow the course of the reaction. The retention time of the desired product is 2.94 minutes, and that of the starting material is 5.25 minutes, at a flow rate of 1 ml/min.

The reaction mixture was poured into 70 ml of deionized water in a separatory funnel, 150 ml of diethyl ether was added, and the mixture carefully shaken. The aqueous layer was extracted again with additional diethyl ether, and was neutralized to pH 6.5 with concentrated ammonium hydroxide, taking care to keep the solution below 30°. It was then extracted a third time with diethyl ether, and the aqueous layer was concentrated to a white powder under vacuum at 30° or less.

The residue was stirred with 50 ml of methanol for one hour, and was filtered. The filtrate was then evaporated to a solid under vacuum, and the solid was taken up in a minimum amount of water and was chromatographed on a 3 x 25 cm column of DIAION HP-20 resin (Mitsubishi), eluting with deionized water. The product-containing fractions were combined and concentrated to a solid under vacuum. The partially desalted solid was slurried with 10 ml of methanol for 10 minutes, and then filtered. The methanol filtrate was concentrated under vacuum to a solid, and that solid was slurried in absolute ethanol at about 100

mg/ml at ambient temperature. The product does not dissolve in ethanol, and was therefore found in the solids upon filtration. Repetition of the slurrying in methanol and ethanol produced 414 mg of the desired product.

The product was identified by fast atom bombardment mass spectroscopy (FABMS), which showed an $(M+1)^+$ of weight 344, and by nuclear magnetic resonance (nmr) analysis in $CD_3OD$ + 10% $D_2O$, which showed features at δ 8.2 (d, H-6), 6.25 (d, H-5), 6.0 (m, H-1′), 4.4 (m, H-3′), 4.1-4.3 (2m, H-4′ and 2H-5′).

Preparation 1

2′-Deoxy-2′,2′-difluoro-5′-O-(hydroxy-4-morpholinylphosphinyl)cytidine complexed with N,N′-methanetetrayl-bis(cyclohexanamine)morpholine(1:1:1)

To a solution of 100 mg of the product of Example 1 in 3 ml of deionized water was added 0.1 ml of morpholine in 3 ml of dry t-butanol. The solution was heated to reflux, and 0.24 g of dicyclohexylcarbodiimide in 4.3 ml of dry t-butanol was added with a syringe pump over a 4 hour period. Another 0.2 ml of morpholine and 24 mg of dicyclohexylcarbodiimide was then added, and the reflux was continued for 1.5 hours more. The mixture was then cooled and let stand overnight. The mixture was then filtered, and the filtrate was concentrated under vacuum. The residue was dissolved in water and extracted several times with diethyl ether, and the aqueous layer was then concentrated under vacuum again. The residue was dissolved by adding 2 ml of methanol and was triturated with anhydrous diethyl ether, and the white solid which resulted was then collected by filtration and dried under vacuum to give 177 mg of the desired product, an amorphous and hygroscopic solid. In FABMS, it showed $(M+1)^+$ of weight 706 (activated nucleoside) and 998 (complex); in TLC on silica gel with 20:15:1.5 n-propanol:$NH_4OH$:water, its $R_f$ was 0.5; and in nmr analysis in $D_2O$ at 300 mHz, it showed the following features: δ 7.85 (d, 1H); 6.25 (t, 1H); 6.14 (d, 1H); 4.45 (m, 1H); 4.20 (m, 1H); 4.10 (m, 1H); 3.94 (m, 1H); 3.90-3.70 (m, 7H); 3.60 (m, 1H); 3.50-3.30 (m, 7H); 3.10 (m, 3H); 2.00-1.60 (m, 12H); 1.45-1.10 (m, 12H).

Example 2

2′-Deoxy-2′,2′-difluorocytidine, 5′-[P′-L-(2,3-bis[palmitoyloxy]propyl)diphosphate

Two g of L-2,3-bis(palmitoyloxy)propylphosphoric acid and 2.3 g of the product of Preparation 1 were added to a 200 ml flask, with 40 ml of distilled pyridine. The solution was then concentrated to dryness under vacuum, and 40 ml of additional pyridine was added. The mixture was stirred briefly, and was again concentrated under vacuum. The dry powder was then redissolved in 75 ml of pyridine and allowed to stand at ambient temperature for four days, after which time the solvent was removed under vacuum and the residue was held under high vacuum for two hours. The solid was then redissolved in 250 ml of 1:1 chloroform:methanol to which approximately 100 ml of 0.1N hydrochloric acid was added. The mixture was stirred briefly, and the organic phase was removed. The aqueous phase was washed twice with additional 1:1 chloroform:methanol, and the combined organic phases were carefully washed with cold water. The organic phase was then concentrated under vacuum to obtain 3.17 g of crude product, which was purified by flash chromatography on an 8 x 8 cm silica gel column, prepared in chloroform. The column was eluted with 9-8:1 chloroform:methanol to remove impurities, and clean product was obtained by eluting with 7-6.5:1 chloroform:methanol. The yield of the product was 1.15 g. The elemental analysis of the desired product was as follows, calculated for $C_{44}H_{79}N_3F_2O_{14}P_2 \cdot 2H_2O$.

| | | | |
|---|---|---|---|
| Theoretical: | C, 52.27; | H, 8.21; | N, 4.15 |
| Found: | C, 51.64; | H, 7.80; | N, 4.13. |

Its $R_f$ in TLC on silica gel, with 25:15:4:2 chloroform:methanol:water:acetic acid, was 0.63.

Example 3

2′-Deoxy-2′,2′-difluorocytidine, 5′-(dihydrogen diphosphate), P′-(2-ethoxyeicosyl) ester

A 127 mg portion of phosphoric acid, mono(2-ethoxyeicosyl) ester and 235 mg of the product of Preparation 1 were added to a 50 ml flask with 5 ml of dry pyridine. The process of Example 2 was

followed to obtain 95 mg of the desired product, which was analyzed by FD mass spectroscopy, showing $(M+1)^+$ of weight 264 (nucleoside) and 312 $(CH_3(CH_2)_{17}CH(OC_2H_5)CH_2)^+$.

Representative compounds of the invention have been tested using murine tumors in female mice to determine their antineoplastic efficacy. Several different tumor systems have been used in these tests, and the compounds have been administered on a number of different dosage schedules. In general, all the tests were started by inoculating female mice with the tumor, subcutaneously. After some number of days delay, intraperitoneal administration of the test compound was begun. Each treatment group consisted of 10 mice, and untreated control groups were included in all experiments. At the completion of the treatment period, the tumors of the control and the treated mice were measured and the mass of them calculated from the measurements. The data are reported here as percent inhibition of tumor growth, based on the tumors of the untreated control mice. The number of animals in each treatment group which exhibited signs of toxicity is also reported.

Test 1

The tumor in this test was M-5 ovarian carcinoma; the compounds were administered as a single dose, two days after inoculation, and the tumors were measured 13 days after inoculation.

| Compound | Dose | Inhibition | Toxicity |
|---|---|---|---|
| Ex. 2 | 50 mg/kg | 87% | 4/10 |
| Ex. 2 | 25 | 72 | 0/10 |
| Ex. 3 | 100 | 64 | 0/10 |
| Ex. 3 | 50 | 53 | 0/10 |

Test 2

The compound of Example 2 was tested against M-5 ovarian carcinoma. Three doses were administered on days 5, 10 and 13 after inoculation, and the tumors were measured on day 15.

| Dose | Inhibition | Toxicity |
|---|---|---|
| 30 mg/kg | 99% | 2/10 |
| 15 | 92 | 0/10 |
| 7.5 | 61 | 0/10 |
| 3.8 | 55 | 0/10 |
| 1.8 | 22 | 0/10 |

Test 3

The compound of Example 2 was tested against M-5 ovarian carcinoma, giving a daily dose from day 5 to day 14 after inoculation. The tumors were measured on day 15.

| Dose | Inhibition | Toxicity |
|---|---|---|
| 10 mg/kg | -- | 10/10 |
| 5 | 100% | 0/10 |
| 2.5 | 69 | 0/10 |
| 1.2 | 58 | 0/10 |
| 0.6 | 42 | 0/10 |

Test 4

The compound of Example 2 was tested against 6C3HED lymphosarcoma, giving a dose on days 1, 5 and 9 after inoculation, and measuring the tumors on day 10.

| Dose | Inhibition | Toxicity |
|---|---|---|
| 30 mg/kg | 98% | 7/10 |
| 15 | 60 | 0/10 |
| 7.5 | 47 | 0/10 |
| 3.8 | 32 | 0/10 |
| 1.8 | 21 | 0/10 |

Test 5

The compound of Example 2 was tested against colon carcinoma 26, giving doses on days 3, 8 and 11 after inoculation, and measuring the tumors on day 13.

| Dose | Inhibition | Toxicity |
|---|---|---|
| 30 mg/kg | 89% | 8/10 |
| 15 | 66 | 0/10 |
| 7.5 | 28 | 0/10 |
| 3.8 | 21 | 0/10 |
| 1.8 | 0 | 0/10 |

Test 6

The compound of Example 2 was tested against X-5563 plasma cell myeloma, giving doses on days 1, 5 and 9 after inoculation and measuring the tumors on day 11.

| Dose | Inhibition | Toxicity |
|---|---|---|
| 30 mg/kg | 100% | 9/10 |
| 15 | 84 | 1/10 |
| 7.5 | 68 | 0/10 |
| 3.8 | 2 | 0/10 |
| 1.8 | 0 | 0/10 |

Test 7

The compound of Example 2 was tested against C3H mammary adenocarcinoma, giving does on days 1, 5 and 9 after inoculation and measuring the tumors on day 11.

| Dose | Inhibition | Toxicity |
|---|---|---|
| 30 mg/kg | 100% | 6/10 |
| 15 | 60 | 0/10 |
| 7.5 | 22 | 0/10 |
| 3.8 | 0 | 0/10 |
| 1.8 | 0 | 0/10 |

Test 8

The compound of Example 2 was tested against Madison lung carcinoma, giving doses on days 1, 5 and 9 after inoculation and measuring the tumors on day 11.

| Dose | Inhibition | Toxicity |
|---|---|---|
| 30 mg/kg | 76% | 4/10 |
| 15 | 68 | 0/10 |
| 7.5 | 33 | 0/10 |
| 3.8 | 18 | 0/10 |
| 1.8 | 12 | 0/10 |

Test 9

The compound of Example 2 was tested against Lewis lung carcinoma, giving doses on days 1, 5 and 9 after inoculation and measuring the tumors on day 11.

| Dose | Inhibition | Toxicity |
|---|---|---|
| 30 mg/kg | 41% | 4/10 |
| 15 | 0 | 0/10 |
| 7.5 | 0 | 0/10 |
| 3.8 | 0 | 0/10 |
| 1.8 | 6 | 0/10 |

The present invention provides a method of treating susceptible neoplasms in mammals comprising administering to a mammal in need of such treatment a pharmaceutically effective amount of a compound of formula I. The method comprises administering the compound to the mammal by various routes including the oral, rectal, transdermal, subcutaneous, intravenous, intramuscular or intranasal routes, preferably orally, subcutaneously, intravenously or intramuscularly.

The term "pharmaceutically effective amount", as defined herein, refers to an appropriate amount of a compound of formula I which is capable of providing chemotherapy to mammals. The active compounds are effective over a wide dosage range. For example, dosages per day will normally fall within the range of about 5 to about 500 $mg/m^2$ of body surface. In the treatment of adult humans, the range of about 10 to about 50 $mg/m^2$, in single or divided doses, is preferred. However, it will be understood that the amount of compound actually administered will be determined by a physician, in the light of the relevant circumstances including the condition to be treated, the particular compound to be administered, the chosen route of administration, the age, weight, and response of the individual patient, and the severity of the patient's symptoms, and therefore the above dosage ranges are not intended to limit the scope of the invention in any way.

The term "susceptible neoplasm", as defined herein, represents an abnormal growth of tissue in mammals capable of being treated by a compound of formula I. While the compounds of formula I are effective against tumors, both solid and non-solid type, the compounds are effective in controlling the growth of rapidly dividing cells because of the compounds' cytotoxic nature. It is a special feature of these compounds that they have a broad spectrum of activity, and are accordingly useful against a variety of tumors, including carcinomas, adenocarcinomas, leukemias, melanomas, sarcomas, lymphosarcomas, and myelomas.

The compounds of the present method are preferably administered as a pharmaceutical formulation. Therefore, as yet another embodiment of the present invention, a pharmaceutical formulation useful for treating susceptible neoplasms in mammals is provided comprising a compound of formula I in combination with a pharmaceutical carrier therefor.

The active ingredient will be present in the formulation in the range of about 1% to about 90% by weight. The active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for

the active ingredient. Thus, the compositions can be in the form of tablets, pills, powders, lozenges, sachets, cachets, elixirs, suspensions, emulsions, solutions, syrups, soft and hard gelatin capsules, suppositories, sterile injectable solutions and sterile packaged powders.

Some examples of suitable carriers include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick or sustained release of the active ingredient after administration to the patient by employing procedures well known in the art.

The compositions are preferably formulated in a unit dosage form, each dosage containing from about 5 to about 500 mg, more usually about 25 to about 300 mg, of the active ingredient, The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

The following formulation examples represent specific pharmaceutical formulations employing compounds comprehended by the present method. The formulations may employ as active compounds any of the compounds of Formula I. The examples are illustrative only and are not intended to limit the scope of the invention in any way.

Formulation 1

Hard gelatin capsules are prepared using the following ingredients:

| | Quantity (mg/capsule) |
|---|---|
| Example 2 | 250 |
| Starch dried | 200 |
| Magnesium stearate | 10 |

The above ingredients are mixed and filled into hard gelatin capsules in 460 mg quantities.

Formulation 2

A tablet formula is prepared using the ingredients below:

| | Quantity (mg/tablet) |
|---|---|
| Example 3 | 250 |
| Cellulose, microcrystalline | 400 |
| Silicon dioxide, fumed | 10 |
| Stearic acid | 5 |

The components are blended and compressed to form tablets each weighing 665 mg.

Formulation 3

An aerosol solution is prepared containing the following components:

| | Weight % |
|---|---|
| Compound 4, Table I | 0.25 |
| Ethanol | 29.75 |
| Propellant 22 (Chlorodifluoromethane) | 70.00 |

The active compound is mixed with ethanol and the mixture added to a portion of the propellant 22, cooled to -30 °C and transferred to a filling device. The required amount is then placed in a stainless steel

container and diluted with the remainder of the propellant. The valve units are then fitted to the container.

Formulation 4

Tablets each containing 60 mg of active ingredient are made up as follows:

| | |
|---|---|
| Compound 7, Table I | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |

The difluoronucleoside, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules each containing 80 mg of medicament are made as follows:

| | |
|---|---|
| Compound 12, Table I | 80 mg |
| Starch | 59 mg |
| Microcrystalline cellulose | 59 mg |
| Magnesium stearate | 2 mg |

The active ingredient, cellulose, starch and magnesium stearate are blended, passed through a No. 45 mesh U.S. sieve, and filled into hard gelatin capsules in 200 mg quantities.

Formulation 6

Suppositories each containing 225 mg of medicament are made as follows:

| | |
|---|---|
| Compound 14, Table I | 225 mg |
| Saturated fatty acid glycerides to | 2 g |

The nucleoside is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

Suspensions each containing 50 mg of medicament per 5 ml dose are made as follows:

| | |
|---|---|
| Compound 16, Table I | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to | 5 ml |

The medicament is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

Formulation 8

An intravenous formulation is prepared as follows:

| | |
|---|---|
| Compound 19, Table I | 100 mg |
| Isotonic saline | 1000 ml |

The solution of the above ingredients is administered intravenously at a rate of 1 ml/minute to a mammal in need of treatment from susceptible neoplasms.

## Claims

**Claims for the following Contracting States : AT, BE, CH, DE, FR, GB, IT, LI, NL, SE**

**1.** A compound of the formula

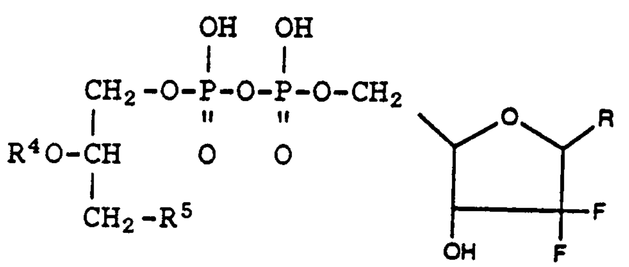

I

wherein R is a base of one of the formulae

A. 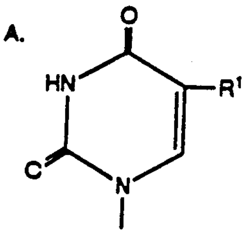

B. 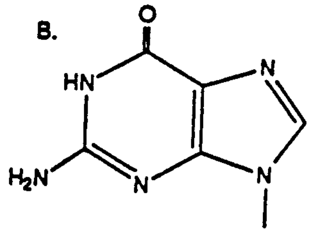

C. 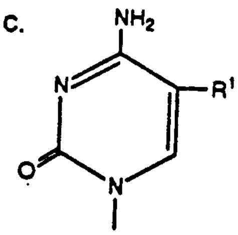

D. 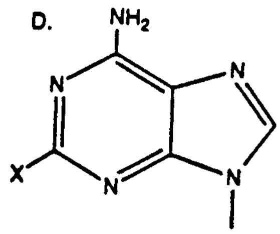

E. 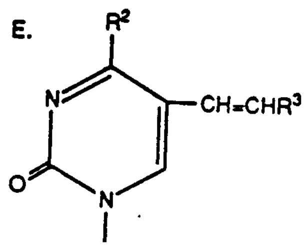

$R^1$ is hydrogen, $C_1$-$C_3$ alkyl, bromo, fluoro, chloro or iodo;
$R^2$ is hydroxy or amino;
$R^3$ is hydrogen, bromo, chloro or iodo;
X is hydrogen, amino, hydroxy, bromo, fluoro or chloro;
$R^4$ is $C_1$-$C_3$ alkyl or $R^6$;
$R^6$ is $CO-(CH_2)_mCH_3$;
m is an integer from 12 through 18;
$R^5$ is $(CH_2)_nCH_3$ or $R^7$;
$R^7$ is $O-CO-(CH_2)_mCH_3$;
n is an integer from 14 through 20;
provided that $R^4$ is $C_1$-$C_3$ alkyl when $R^5$ is $(CH_2)_nCH_3$;and that $R^4$ is $R^6$ when $R^5$ is $R^7$;
or a pharmaceutically acceptable salt thereof.

2. A compound of Claim 1 wherein R is of formula C and $R^1$ is hydrogen.

3. A compound of either of Claims 1 or 2 which is in the $\beta$ form.

4. The compound of Claim 1 which is 2′-deoxy-2′,2′-difluorocytidine-5′-[P′-L-(2,3-bis[palmitoyloxy]propyl)-diphosphate or a pharmaceutically acceptable salt thereof.

5. The compound of Claim 1 which is 2′-deoxy-2′,2′-difluorocytidine-5′-(dihydrogen-diphosphate)-P′-(2-ethoxyeicosyl) ester or a pharmaceutically acceptable salt thereof.

6. A pharmaceutical formulation comprising a pharmaceutical carrier and a compound as claimed in any one of Claims 1 to 5.

7. A compound as claimed in any one of Claims 1 to 5 for use as a pharmaceutical.

**8.** A compound of the formula

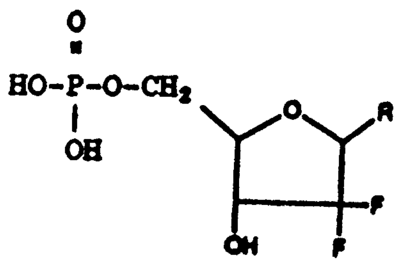

II

wherein R is as defined in Claim 1; provided that when R is of formula C, $R^1$ is not hydrogen; or a pharmaceutically acceptable salt thereof.

**9.** A process for preparing a compound of Formula I, as defined in any one of Claims 1 to 5, comprising reacting a compound of Formula II, as defined in either Claim 8 or 9, with a compound of the formula

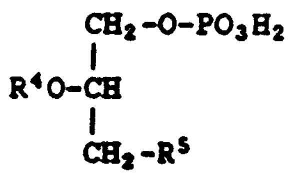

IV

wherein $R^4$ and $R^5$ are as defined in any one of Claims 1 to 5.

**Claims for the following Contracting State : ES**

**1.** A process for preparing a compound of the formula

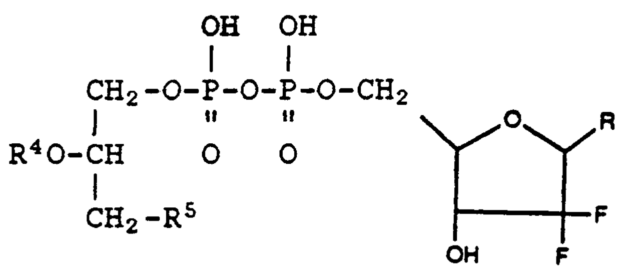

I

wherein R is a base of one of the formulae

A.

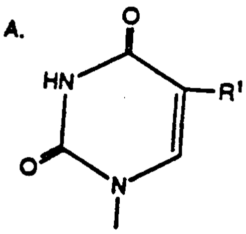

B.

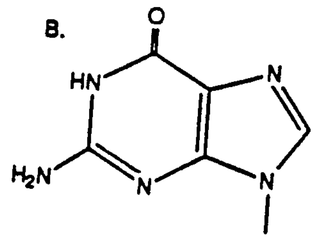

C.

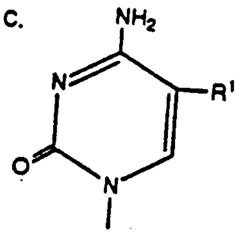

D.

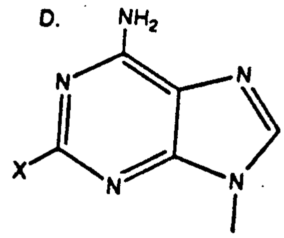

E.

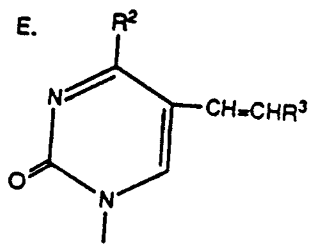

$R^1$ is hydrogen, $C_1$-$C_3$ alkyl, bromo, fluoro, chloro or iodo;
$R^2$ is hydroxy or amino;
$R^3$ is hydrogen, bromo, chloro or iodo;
X is hydrogen, amino, hydroxy, bromo, fluoro or chloro;
$R^4$ is $C_1$-$C_3$ alkyl or $R^6$;
$R^6$ is $CO$-$(CH_2)_mCH_3$;
m is an integer from 12 through 18;
$R^5$ is $(CH_2)_nCH_3$ or $R^7$;
$R^7$ is $O$-$CO$-$(CH_2)_mCH_3$;
n is an integer from 14 through 20;

provided that $R^4$ is $C_1$-$C_3$ alkyl when $R^5$ is $(CH_2)_nCH_3$;and that $R^4$ is $R^6$ when $R^5$ is $R^7$; or a pharmaceutically acceptable salt thereof.

**2.** A process of Claim 1 wherein R is of formula C and $R^1$ is hydrogen.

**3.** A process of either Claim 1 or 2 wherein the product is in the $\beta$ form.

**4.** The process of either Claim 2 or 3 for preparing 2′-deoxy-2′,2′-difluorocytidine-5′-[P′-L-(2,3-bis-[palmitoyloxy]propyl)diphosphate or a pharmaceutically acceptable salt thereof.

**5.** The process of either Claim 2 or 3 for preparing 2′-deoxy-2′,2′-difluorocytidine-5′-(dihydrogen diphosphate)-P′-(2-ethoxyeicosyl) ester or a pharmaceutically acceptable salt thereof.

**6.** A process for preparing a pharmaceutical formulation comprising admixing a pharmaceutical carrier and a compound as claimed in any one of Claims 1 to 5.

**Claims for the following Contracting State : GR**

**1.** A process for preparing a compound of the formula

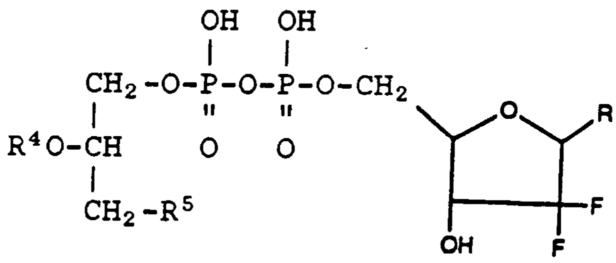

I

wherein R is a base of one of the formulae

A.

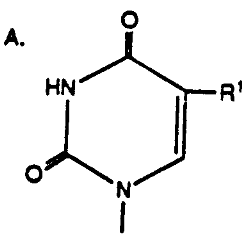

B.

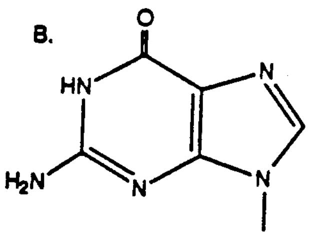

C.

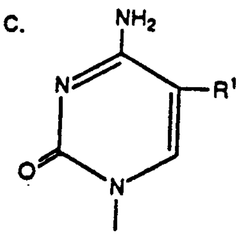

D.

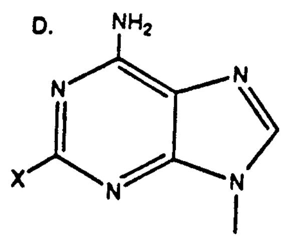

E.

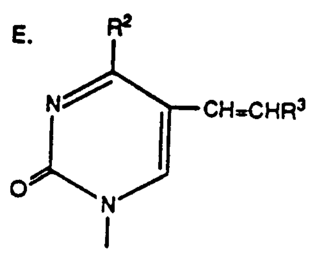

$R^1$ is hydrogen, $C_1$-$C_3$ alkyl, bromo, fluoro, chloro or iodo;
$R^2$ is hydroxy or amino;
$R^3$ is hydrogen, bromo, chloro or iodo;
X is hydrogen, amino, hydroxy, bromo, fluoro or chloro;
$R^4$ is $C_1$-$C_3$ alkyl or $R^6$;
$R^6$ is $CO$-$(CH_2)_mCH_3$;
m is an integer from 12 through 18;
$R^5$ is $(CH_2)_nCH_3$ or $R^7$;
$R^7$ is $O$-$CO$-$(CH_2)_mCH_3$;
n is an integer from 14 through 20;
provided that $R^4$ is $C_1$-$C_3$ alkyl when $R^5$ is $(CH_2)_nCH_3$;and that $R^4$ is $R^6$ when $R^5$ is $R^7$;
or a pharmaceutically acceptable salt thereof.

**2.** A process of Claim 1 wherein R is of formula C and $R^1$ is hydrogen.

**3.** A process of either Claim 1 or 2 wherein the product is in the $\beta$ form.

**4.** The process of either Claim 2 or 3 for preparing 2'-deoxy-2',2'-difluorocytidine-5'-[P'-L-(2,3-bis-[palmitoyloxy]propyl)diphosphate or a pharmaceutically acceptable salt thereof.

**5.** The process of either Claim 2 or 3 for preparing 2'-deoxy-2',2'-difluorocytidine-5'-(dihydrogen diphosphate)-P'-(2-ethoxyeicosyl) ester or a pharmaceutically acceptable salt thereof.

**6.** A process for preparing a pharmaceutical formulation comprising admixing a pharmaceutical carrier and a compound as claimed in any one of Claims 1 to 5.

**7.** A compound of the formula

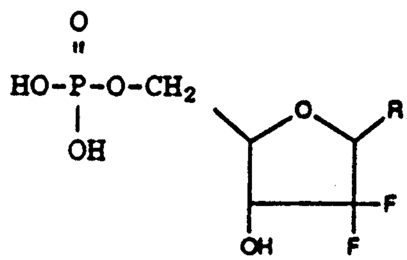

II

wherein R is as defined in Claim 1; provided that when R is of formula C, $R^1$ is not hydrogen; or a pharmaceutically acceptable salt thereof.

**Patentansprüche**
**Patentansprüche für folgende Vertragsstaaten : AT, BE, CH, DE, FR, GB, IT, LI, NL, SE**

**1.** Verbindung der Formel

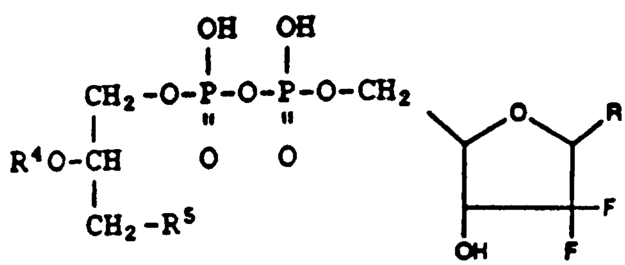

I

worin
R eine Base gemäß einer der Formeln

A.
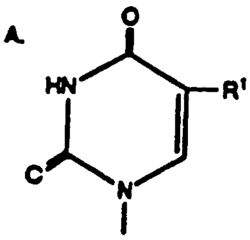

B.
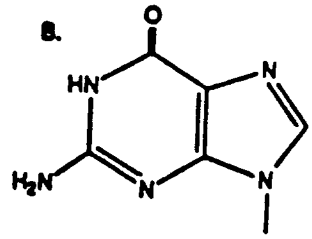

C.
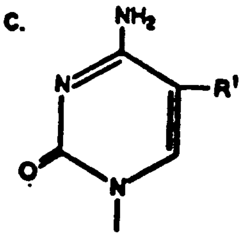

D.
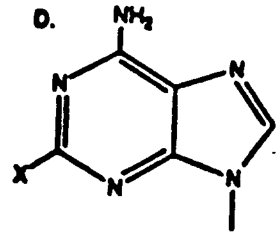

E.
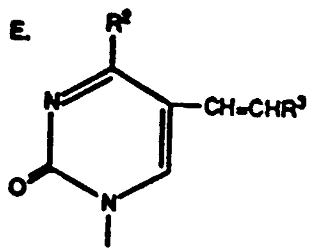

ist,

| | |
|---|---|
| $R^1$ | Wasserstoff, ein $C_1$-$C_3$-Alkylrest, Brom, Fluor, Chlor oder Iod ist; |
| $R^2$ | ein Hydroxy- oder Aminorest ist; |
| $R^3$ | Wasserstoff, Brom, Chlor oder Iod ist; |
| X | Wasserstoff, ein Amino-, Hydroxyrest, Brom, Fluor oder Chlor ist; |
| $R^4$ | ein $C_1$-$C_3$-Alkylrest oder $R^6$ ist; |
| $R^6$ | $CO-(CH_2)_mCH_3$ ist; |
| m | eine ganze Zahl von 12 bis 18 ist; |
| $R^5$ | $(CH_2)_nCH_3$ oder $R^7$ ist; |
| $R^7$ | $O-CO-(CH_2)_mCH_3$ ist; |
| n | eine ganze Zahl von 14 bis 20 ist; |

mit dem Vorbehalt, daß $R^4$ ein $C_1$-$C_3$-Alkylrest ist, wenn $R^5$ $(CH_2)_nCH_3$ ist; und daß $R^4$ $R^6$ ist, wenn $R^5$ $R^7$ ist;
oder ein pharmazeutisch annehmbares Salz davon.

**2.** Verbindung nach Anspruch 1, worin R die Formel C hat und $R^1$ Wasserstoff ist.

**3.** Verbindung nach einem der Ansprüche 1 oder 2, die in der $\beta$-Form ist.

**4.** Verbindung nach Anspruch 1, die 2'-Deoxy-2',2'-difluorcytidin-5'-[P'-L-(2,3-bis[palmitoyloxy]propyl)-diphosphat] oder ein pharmazeutisch annehmbares Salz davon ist.

**5.** Verbindung nach Anspruch 1, die 2'-Deoxy-2',2'-difluorcytidin-5'-(dihydrogendiphosphat)-P'-(2-ethoxyeicosyl)ester oder ein pharmazeutisch annehmbares Salz davon ist.

**6.** Pharmazeutisches Präparat, umfassend einen pharmazeutischen Träger und eine Verbindung nach einem der Ansprüche 1 bis 5.

**7.** Verbindung nach einem der Ansprüche 1 bis 5 zur Verwendung als Pharmazeutikum.

**8.** Verbindung der Formel

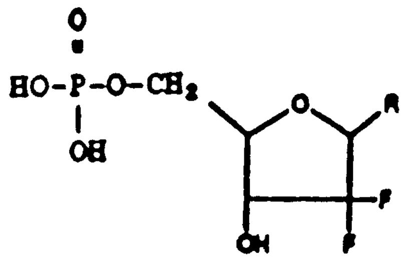

II

worin R wie in Anspruch 1 definiert ist, mit dem Vorbehalt, daß dann, wenn R die Formel C hat, $R^1$ nicht Wasserstoff ist; oder ein pharmazeutisch annehmbares Salz davon.

**9.** Verfahren zur Herstellung einer Verbindung der Formel I nach einem der Ansprüche 1 bis 5, umfassend, daß man eine Verbindung der Formel II, wie in einem der Ansprüche 8 oder 9 definiert, mit einer Verbindung der Formel

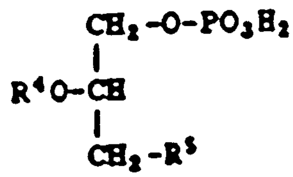

IV

worin $R^4$ und $R^5$ wie in einem der Ansprüche 1 bis 5 definiert sind, umsetzt.

**Patentansprüche für folgenden Vertragsstaat : ES**

**1.** Verfahren zur Herstellung einer Verbindung der Formel

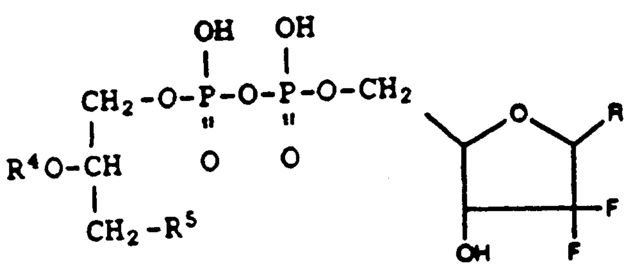

I

worin
R eine Base gemäß einer der Formeln

A.

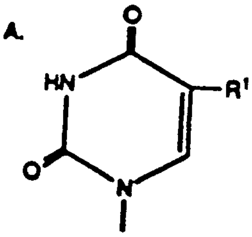

B.

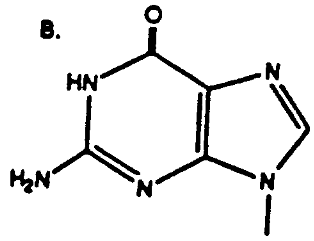

C.

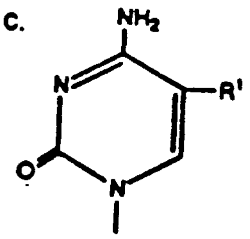

D.

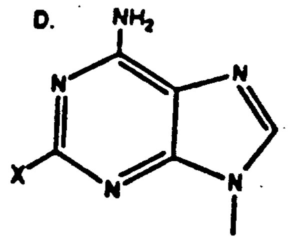

E.

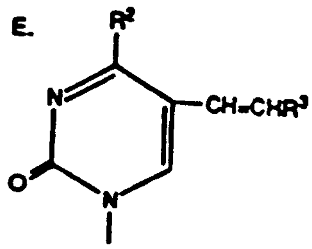

ist,

$R^1$ Wasserstoff, ein $C_1$-$C_3$-Alkylrest, Brom, Fluor, Chlor oder Iod ist;
$R^2$ ein Hydroxy- oder Aminorest ist;
$R^3$ Wasserstoff, Brom, Chlor oder Iod ist;
X Wasserstoff, ein Amino-, Hydroxyrest, Brom, Fluor oder Chlor ist;
$R^4$ ein $C_1$-$C_3$-Alkylrest oder $R^6$ ist;
$R^6$ $CO$-$(CH_2)_mCH_3$ ist;
m eine ganze Zahl von 12 bis 18 ist;
$R^5$ $(CH_2)_nCH_3$ oder $R^7$ ist;
$R^7$ $O$-$CO$-$(CH_2)_mCH_3$ ist;
n eine ganze Zahl von 14 bis 20 ist;

mit dem Vorbehalt, daß $R^4$ ein $C_1$-$C_3$-Alkylrest ist, wenn $R^5$ $(CH_2)_nCH_3$ ist; und daß $R^4$ $R^6$ ist, wenn $R^5$ $R^7$ ist;
oder ein pharmazeutisch annehmbares Salz davon.

**2.** Verfahren nach Anspruch 1, worin R die Formel C hat und $R^1$ Wasserstoff ist.

**3.** Verfahren nach einem der Ansprüche 1 oder 2, worin das Produkt in der $\beta$-Form ist.

**4.** Verfahren nach einem der Ansprüche 2 oder 3 zur Herstellung von 2'-Deoxy-2',2'-difluorcytidin-5'-[P'-L-(2,3-bis[palmitoyloxy]propyl)diphosphat] oder eines pharmazeutisch annehmbaren Salzes davon.

**5.** Verfahren nach einem der Ansprüche 2 oder 3 zur Herstellung von 2'-Deoxy-2',2'-difluorcytidin-5'-(dihydrogendiphosphat)-P'-(2-ethoxyeicosyl)ester oder eines pharmazeutisch annehmbaren Salzes davon.

**6.** Verfahren zur Herstellung eines pharmazeutischen Präparates, umfassend, daß man einen pharmazeutischen Träger und eine Verbindung nach einem der Ansprüche 1 bis 5 vermischt.

**Patentansprüche für folgenden Vertragsstaat : GR**

**1.** Verfahren zur Herstellung einer Verbindung der Formel

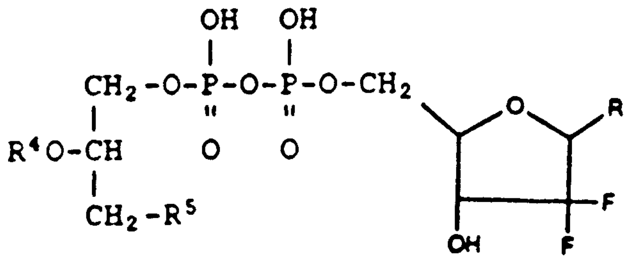

I

worin

R eine Base gemäß einer der Formeln

A.

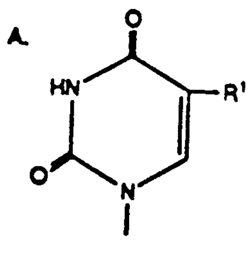

B.

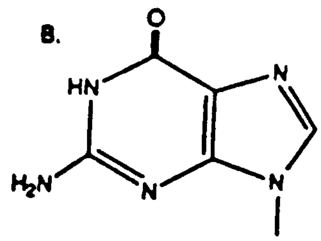

C.

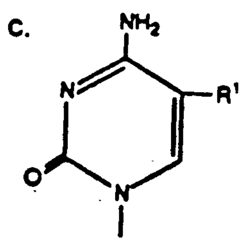

D.

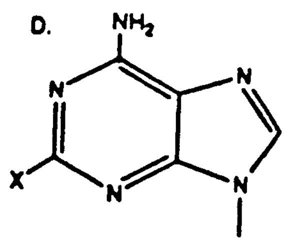

E.

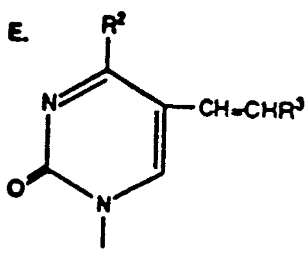

ist,

$R^1$ Wasserstoff, ein $C_1$-$C_3$-Alkylrest, Brom, Fluor, Chlor oder Iod ist;

$R^2$ ein Hydroxy- oder Aminorest ist;

$R^3$ Wasserstoff, Brom, Chlor oder Iod ist;

X Wasserstoff, ein Amino-, Hydroxyrest, Brom, Fluor oder Chlor ist;

$R^4$ ein $C_1$-$C_3$-Alkylrest oder $R^6$ ist;

$R^6$ $CO-(CH_2)_mCH_3$ ist;

m eine ganze Zahl von 12 bis 18 ist;

$R^5$ $(CH_2)_nCH_3$ oder $R^7$ ist;

$R^7$ $O-CO-(CH_2)_mCH_3$ ist;

n eine ganze Zahl von 14 bis 20 ist;
mit dem Vorbehalt, daß $R^4$ ein $C_1$-$C_3$-Alkylrest ist, wenn $R^5$ $(CH_2)_nCH_3$ ist; und daß $R^4$ $R^6$ ist, wenn $R^5$ $R^7$ ist;
oder ein pharmazeutisch annehmbares Salz davon.

**2.** Verfahren nach Anspruch 1, worin R die Formel C hat und $R^1$ Wasserstoff ist.

**3.** Verfahren nach einem der Ansprüche 1 oder 2, worin das Produkt in der $\beta$-Form ist.

**4.** Verfahren nach einem der Ansprüche 2 oder 3 zur Herstellung von 2'-Deoxy-2',2'-difluorcytidin-5'-[P'-L-(2,3-bis[palmitoyloxy]propyl)diphosphat] oder eines pharmazeutisch annehmbaren Salzes davon.

**5.** Verfahren nach einem der Ansprüche 2 oder 3 zur Herstellung von 2'-Deoxy-2',2'-difluorcytidin-5'-(dihydrogendiphosphat)-P'-(2-ethoxyeicosyl)ester oder eines pharmazeutisch annehmbaren Salzes davon.

**6.** Verfahren zur Herstellung eines pharmazeutischen Präparates, umfassend, daß man einen pharmazeutischen Träger und eine Verbindung nach einem der Ansprüche 1 bis 5 vermischt.

**7.** Verbindung der Formel

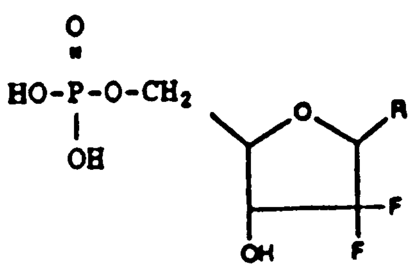

II

worin R wie in Anspruch 1 definiert ist, mit dem Vorbehalt, daß dann, wenn R die Formel C hat, $R^1$ nicht Wasserstoff ist; oder ein pharmazeutisch annehmbares Salz davon.

**Revendications**
**Revendications pour les Etats contractants suivants : AT, BE, CH, DE, FR, GB, IT, LI, NL, SE**

**1.** Composé répondant à la formule

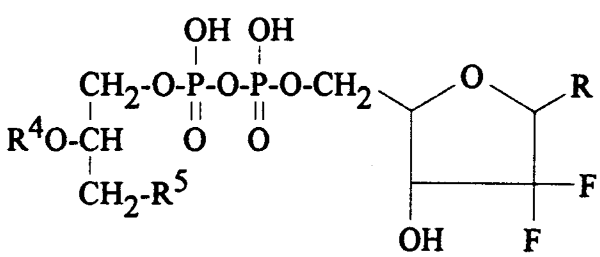

I

dans laquelle R représente une base répondant à une des formules

A.

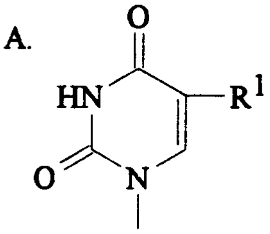

B.

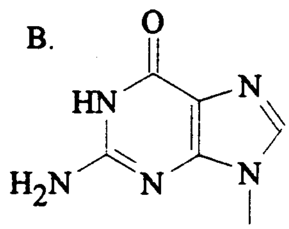

C.

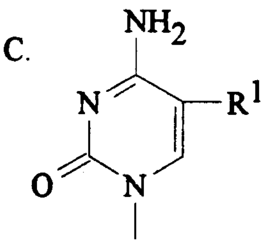

D.

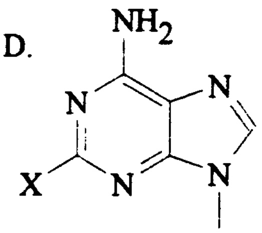

E.

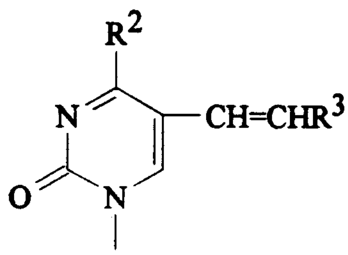

$R^1$ représente un atome d'hydrogène, un groupe alkyle en $C_1$-$C_3$, un atome de brome, un atome de fluor, un atome de chlore ou un atome d'iode;

$R^2$ représente un groupe hydroxyle ou un groupe amino;

$R^3$ représente un atome d'hydrogène, un atome de brome, un atome de chlore ou un atome d'iode;

X représente un atome d'hydrogène, un groupe amino, un groupe hydroxyle, un atome de brome, un atome de fluor ou un atome de chlore;

$R^4$ représente un groupe alkyle en $C_1$-$C_3$ ou un groupe $R^6$;

$R^6$ représente un groupe $CO$-$(CH_2)_mCH_3$;

m représente un nombre entier de 12 à 18;

$R^5$ représente un groupe $(CH_2)_nCH_3$ ou un groupe $R^7$;

$R^7$ représente un groupe $O$-$CO$-$(CH_2)_mCH_3$;

n représente un nombre entier de 14 à 20;

pour autant que $R^4$ représente un groupe alkyle en $C_1$-$C_3$ lorsque $R^5$ représente un groupe $(CH_2)_nCH_3$; et que $R^4$ représente un groupe $R^6$ lorsque $R^5$ représente un groupe $R^7$;
ou un sel pharmaceutiquement acceptable de celui-ci.

**2.** Composé selon la revendication 1, dans lequel R répond à la formule C et $R^1$ représente un atome d'hydrogène.

**3.** Composé selon l'une ou l'autre des revendications 1 ou 2, qui se trouve sous forme $\beta$.

**4.** Composé selon la revendication 1, qui est le 2'-désoxy-2',2'-difluorocytidine-5'-[P'-L-(2,3-bis-[palmitoyloxy]propyl)diphosphate ou un sel pharmaceutiquement acceptable de celui-ci.

**5.** Composé selon la revendication 1, qui est l'ester 2'-désoxy-2',2'-difluorocytidine-5'-(dihydrogénodiphosphate)-P'-(2-éthoxyéicosyle) ou un sel pharmaceutiquement acceptable de celui-ci.

**6.** Formulation pharmaceutique comprenant un support pharmaceutique et un composé selon l'une quelconque des revendications 1 à 5.

7. Composé selon l'une quelconque des revendications 1 à 5 pour l'utilisation comme produit pharmaceutique.

8. Composé répondant à la formule

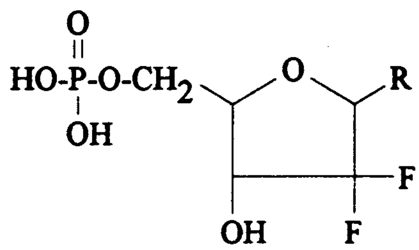

II

dans laquelle R est tel que défini à la revendication 1; pour autant que si R répond à la formule C, $R^1$ n'est pas un atome d'hydrogène; ou un sel pharmaceutiquement acceptable de celui-ci.

9. Procédé pour la préparation d'un composé répondant à la Formule I, selon l'une quelconque des revendications 1 à 5, comprenant la mise en réaction d'un composé répondant à la Formule II, selon l'une ou l'autre des revendications 8 ou 9, avec un composé répondant à la formule

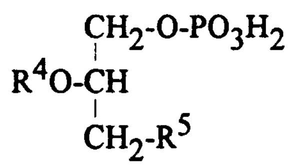

IV

dans laquelle $R^4$ et $R^5$ sont tels que définis dans l'une quelconque des revendications 1 à 5.

**Revendications pour l'Etat contractant suivant : ES**

1. Procédé pour la préparation d'un composé répondant à la formule

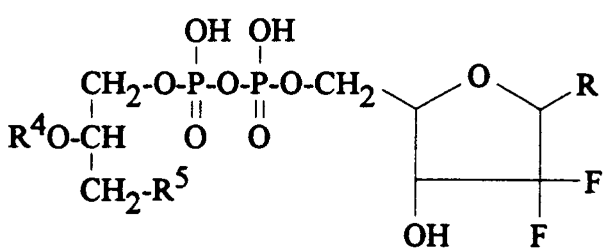

I

dans laquelle R représente une base répondant à une des formules

A.
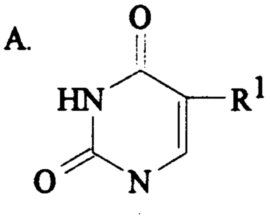

B.
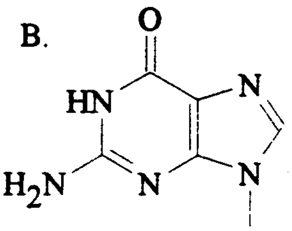

C.
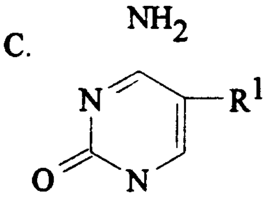

D.
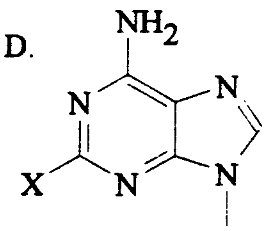

E.
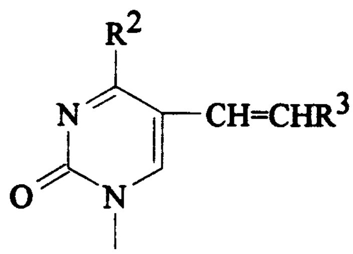

$R^1$ représente un atome d'hydrogène, un groupe alkyle en $C_1$-$C_3$, un atome de brome, un atome de fluor, un atome de chlore ou un atome d'iode;

$R^2$ représente un groupe hydroxyle ou un groupe amino;

$R^3$ représente un atome d'hydrogène, un atome de brome, un atome de chlore ou un atome d'iode;

X représente un atome d'hydrogène, un groupe amino, un groupe hydroxyle, un atome de brome, un atome de fluor ou un atome de chlore;

$R^4$ représente un groupe alkyle en $C_1$-$C_3$ ou un groupe $R^6$;

$R^6$ représente un groupe CO-$(CH_2)_mCH_3$;

m représente un nombre entier de 12 à 18;

$R^5$ représente un groupe $(CH_2)_nCH_3$ ou un groupe $R^7$;

$R^7$ représente un groupe O-CO-$(CH_2)_mCH_3$;

n représente un nombre entier de 14 à 20;

pour autant que $R^4$ représente un groupe alkyle en $C_1$-$C_3$ lorsque $R^5$ représente un groupe $(CH_2)_nCH_3$; et que $R^4$ représente un groupe $R^6$ lorsque $R^5$ représente un groupe $R^7$;
ou d'un sel pharmaceutiquement acceptable de celui-ci.

**2.** Procédé selon la revendication 1, dans lequel R répond à la formule C et $R^1$ représente un atome d'hydrogène.

**3.** Procédé selon l'une ou l'autre des revendications 1 ou 2, dans lequel le produit se trouve sous forme $\beta$.

**4.** Procédé selon l'une ou l'autre des revendications 2 ou 3, pour la préparation du 2'-désoxy-2',2'-difluorocytidine-5'-[P'-L-(2,3-bis[palmitoyloxy]propyl)-diphosphate ou d'un sel pharmaceutiquement acceptable de celui-ci.

**5.** Procédé selon l'une ou l'autre des revendications 2 ou 3, pour la préparation de l'ester 2'-désoxy-2',2'-difluorocytidine-5'-(dihydrogénodiphosphate)-P'-(2-éthoxyéicosyle) ou d'un sel pharmaceutiquement acceptable de celui-ci.

6. Procédé pour la préparation d'une formulation pharmaceutique comprenant le mélange d'un support pharmaceutique et d'un composé selon l'une quelconque des revendications 1 à 5.

**Revendications pour l'Etat contractant suivant : GR**

1. Procédé pour la préparation d'un composé répondant à la formule

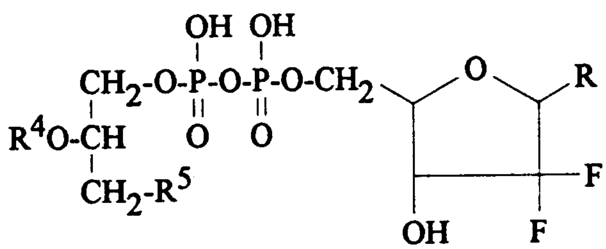

I

dans laquelle R représente une base répondant à une des formules

A. 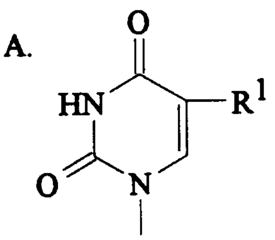

B. 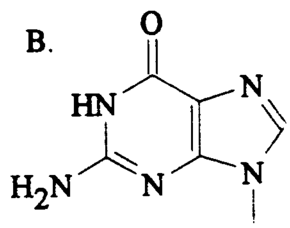

C. 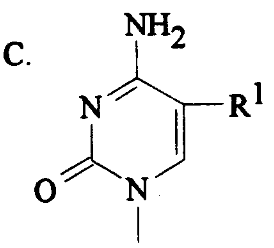

D. 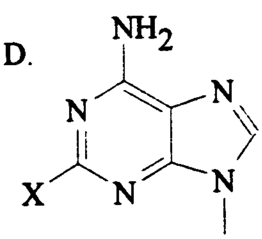

E. 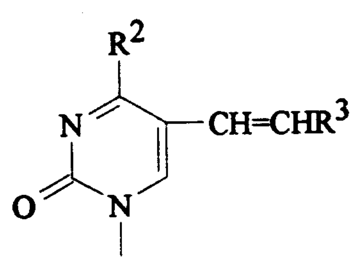

$R^1$ représente un atome d'hydrogène, un groupe alkyle en $C_1$-$C_3$, un atome de brome, un atome de fluor, un atome de chlore ou un atome d'iode;

$R^2$ représente un groupe hydroxyle ou un groupe amino;

$R^3$ représente un atome d'hydrogène, un atome de brome, un atome de chlore ou un atome d'iode;

X représente un atome d'hydrogène, un groupe amino, un groupe hydroxyle, un atome de brome, un atome de fluor ou un atome de chlore;

$R^4$ représente un groupe alkyle en $C_1$-$C_3$ ou un groupe $R^6$;

$R^6$ représente un groupe $CO-(CH_2)_mCH_3$;

m représente un nombre entier de 12 à 18;

$R^5$ représente un groupe $(CH_2)_nCH_3$ ou un groupe $R^7$;

$R^7$ représente un groupe O-CO-$(CH_2)_mCH_3$;
n représente un nombre entier de 14 à 20;
pour autant que $R^4$ représente un groupe alkyle en $C_1$-$C_3$ lorsque $R^5$ représente un groupe $(CH_2)_nCH_3$; et que $R^4$ représente un groupe $R^6$ lorsque $R^5$ représente un groupe $R^7$; ou d'un sel pharmaceutiquement acceptable de celui-ci.

**2.** Procédé selon la revendication 1, dans lequel R répond à la formule C et $R^1$ représente un atome d'hydrogène.

**3.** Procédé selon l'une ou l'autre des revendications 1 ou 2, dans lequel le produit se trouve sous forme $\beta$.

**4.** Procédé selon l'une ou l'autre des revendications 2 ou 3, pour la préparation du 2'-désoxy-2',2'-difluorocytidine-5'-[P'-L-(2,3-bis[palmitoyloxy]propyl)diphosphate ou d'un sel pharmaceutiquement acceptable de celui-ci.

**5.** Procédé selon l'une ou l'autre des revendications 2 ou 3, pour la préparation de l'ester 2'-désoxy-2',2'-difluorocytidine-5'-(dihydrogénodiphosphate)-P'-(2-éthoxyeicosyle) ou d'un sel pharmaceutiquement acceptable de celui-ci.

**6.** Procédé pour la préparation d'une formulation pharmaceutique comprenant le mélange d'un support pharmaceutique et d'un composé selon l'une quelconque des revendications 1 à 5.

**7.** Composé répondant à la formule

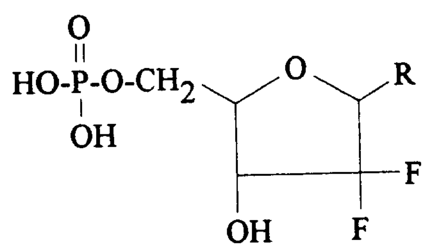

II

dans laquelle R est tel que défini à la revendication 1; pour autant que si R répond à la formule C, $R^1$ n'est pas un atome d'hydrogène; ou un sel pharmaceutiquement acceptable de celui-ci.